# EUROPEAN PATENT SPECIFICATION

(11) **EP 1 638 516 B1**
(45) Date of publication and mention of the grant of the patent: **10.03.2010**
(21) Application number: 04743191.1
(22) Date of filing: 01.07.2004
(51) Int. Cl.: A61Q 17/00, A61K 8/40, A61K 8/41

(54) **BARRIER FORMULATION COMPRISING A SILICONE BASED EMULSION**
BARRIEREFORMULIERUNG MIT EINER EMULSION AUF SILIKONBASIS
FORMULATION CONSTITUANT UNE BARRIERE COMPRENANT UNE EMULSION A BASE DE SILICONE

(30) Priority: 01.07.2003 GB 0315430; 04.07.2003 GB 0315726
(43) Date of publication of application: 29.03.2006
(73) Proprietor: SkinSure International Limited, Park Royal London NW10 7AW (GB)
(72) Inventor: BENNETT, Brian, Nuneaton, Warwickshire CV11 6JQ (GB)
(74) Representative: Ford, Timothy James
(86) International application number: PCT/GB2004/002845
(87) International publication number: WO 2005/002537

(56) References cited:
- EP-A- 0 627 259
- WO-A-01/74983
- WO-A-02/078667
- WO-A-03/034994
- WO-A-03/039401
- US-A- 5 208 013
- US-A- 5 648 083
- US-A- 5 658 559
- US-A- 6 136 771
- US-A1- 2001 016 589
- US-B1- 6 387 382

## Description

The invention relates to an improved barrier formulation and a method for its manufacture.

Barrier creams or lotions are useful in a number of applications. For example, barrier creams or lotions can be useful where protective clothing cannot be used or is not appropriate. This is to solve the problems of contact dermatitis and hospital acquired infection.

US 5648083 describes personal care compositions including water, dimethicone, a polymeric emulsifier and optionally a water-soluble polyol, a pH-adjusting agent, an anti-microbial agent and a chelating agent. This document also describes disposable wipe products containing a non woven or other substrate impregnated with the composition.

US 6387382 describes multipurpose skin preparations in the form of oil-in-water emulsions prepared by combining an oil phase and an aqueous phase. The oil phase comprises a siloxane polymer, a cyclic silicone, an anti-oxidant, and an emollient the aqueous phase comprises at least a humectant, a rheology modifier, a thickener and water. The skin preparation can include, in the oil phase, therapeutic agent such as oat flour or tea tree oil.

US 5208013 describes a composition for topical application which provides a barrier against irritation and painful damage due to contact with many otherwise harmful materials. The composition comprises in combination water, dimethicone, stearic acid, coconut fatty acid, isopropyl myristate, glycerine, triethanolamine, cetyl alcohol, polyvinyl pyrrolidone, cetearyl alcohol, ceteareth-20, tetrasodium EDTA, hydroxyethyl cellulose, aloe vera gel, vitamin E and lanolin. In certain applications such as for use by doctors, dentists and other health professionals, it is advantageous to add a bactericide to the composition.

US5658559 relates to a barrier moisturizing lotion for treating pathologies of the skin, the lotion being composed of an oil-in-water emulsion that includes water, one or more emollients, at least one polyhydric alcohol, a barrier polymer and a therapeutical agent.

EP0627259 discloses a method of manufacturing a formulation comprising an emulsion (oil-in-water emulsion) having at least an oil phase and an aqueous phase wherein the oil phase comprises a silicone compound and which method comprises the step of a) preparing an oil phase containing a silicone compound, b) preparing an aqueous phase, c) mixing the oil phase and the aqueous phase together and d) neutralising the mixture with a neutralising agent.

WO03/034994 discloses a skin formulation in the form of a hydroalcoholic gel, containing a silicone oil, alcohol, water, hydrogel, an emollient, emulsifier, a neutralizing agent, wherein the composition has a viscosity of below 2000 centipoise at between 20-40°C and wherein the composition comprises an antimicrobial agent, i.e. triclosan in an amount of between 0.1-2.0 wt% as well as another active agent. The skin formulation described in document D6 is active against *Staphylococcus aureus.*

US6136771 refers to formulations against *Staphylococcus aureus* containing high concentrations of antimicrobial agents, such as triclosan in an amount of at least 25%.

US2001/0016589 discloses triclosan-based formulations in combination with alexidine against *Staphylococcus aureus.*

Contact dermatitis can arise for workers in certain environments such as hairdressing, health care etc. It is generally caused by substances that come into contact with the skin. A substance that causes allergic contact dermatitis is called an "allergen". If a person is allergic to an allergen, then contact between that allergen and the person's skin can produce itching and blisters (allergic contact dermatitis). Allergic contact dermatitis is not usually caused by irritants such as acid, alkali, solvents, strong soap or detergent. However some chemicals are both irritants and allergens. Examples of known allergens include nickel, rubber, para-phenylene-diamine hair dye, neomycin, chromates, and plant products.

The problem with existing barrier formulations is that they do not last particularly long before losing their properties and have to be frequently re-applied. Therefore they do not provide complete protection against allergens. A way of ameliorating this problem has been sought.

Hospital acquired infection (HAI) is a major cause for concern in the clinical setting. In the UK, it is estimated that approximately 9% of in-patients have a HAI at any one time, which equates to at least 300,000 infections per annum (National Audit Office, "The Management and Control of Hospital Acquired Infection in acute NHS Trusts in England', London: Stationery Office 2000). The costs associated with treating HAI are difficult to measure, however results from a recent study undertaken by the London School of Hygiene and Tropical Medicine and the Central Public Health Laboratory, suggest that HAI may be costing the NHS as much as £1 billion per year (*ibid*). It is not possible to prevent all HAIs, however, the National Audit Office report suggests that HAIs could be reduced by up to 15% through better application of existing knowledge and realistic and effective infection control practices (*ibid*).

Hand hygiene is one of the basic components of any infection control program and is possibly the most important factor in preventing HAI. Indeed, outbreaks originating from a common source have been traced to contaminated hands of health care workers (HCW) (Boyce JM, Potter-Bynoe G, Opal SM et al "A Common-Source Outbreak of Staphylococcus epidermidis Infections among Patients undergoing Cardiac Surgery Journal of Infectious Diseases 1990; 161: 493-499). In a recent survey, it was demonstrated that HCW understand the importance of hand hygiene as a means of preventing infection and are in favour of interventions that make hand hygiene easier (Harris AD, Samore MH, Nafziger R et al "A Survey on Hand Washing Practices and Opinions of Healthcare Workers" Journal of Hospital Infection 2000; 45:318-21). However, studies have also consistently demonstrated that HCW frequently do not wash their hands and compliance rates are low (Pittet D, Mourouga P, Perneger TV "Compliance with Handwashing in a Teaching Hospital" Ann Intern Med 1999; 130: 126-130).

The recent EPIC evidence based guidelines on hand hygiene, commissioned by the Department of Health (Pratt RJ, Pellowe C, Loveday HP et al "The EPIC Project: Developing National Evidence-Based Guidelines for Preventing Healthcare Associated Infections, Phase I: Guidelines for Preventing Hospital Acquired Infections" Journal of Hospital Infection 2001; 47 (suppl): S1-82) recommends that formal hand washing with soap and water should be undertaken by HCW when hands are visibly soiled or contaminated, and alcohol-glycerol hand rub used between patients. These methods of hand hygiene require adequate cleansing facilities, including suitably located sinks and gel dispensers of sufficient numbers, which may not always be present within the clinical setting. In view of the lack of compliance with hand hygiene and despite many initiatives, new ways of ameliorating this problem have been sought.

According to a first aspect of the invention, there is provided a barrier formulation which comprises an emulsion having at least an oil phase comprising a silicone compound and an aqueous phase, the viscosity of the formulation being 20 Pascal second (20000 cps) or less, one or more of an emollient, an excipient, a thickener, an emulsifier, a neutralising agent, a preservative, and water, and the formulation further consisting of triclosan as an active ingredient present in an amount from 0.5 to 10 % by weight of the formulation.

According to a second aspect of the invention, there is also provided a method of manufacturing a formulation comprising an emulsion having at least an oil phase and an aqueous phase wherein the oil phase comprises a silicone compound which method comprises the steps of:
(a) preparing an oil phase containing a silicone compound;
(b) preparing an aqueous phase;
(c) mixing the oil phase and the aqueous phase together;
(d) neutralising the mixture with a neutralising agent.

According to a further aspect of the invention, there is provided a barrier formulation of the invention for use as a skin barrier.

The formulation according to the invention has been found to act as an effective skin barrier to irritants and/or allergens. In particular, it has been found to be possible to apply concentrated sulphuric acid to the skin of a person which has been pre-treated with the formulation according to the invention with no ill effects to the person's skin. Indeed the formulation of the invention has been found to prevent and/or reduce contact dermatitis and other skin conditions in workers such as health care workers, hairdressers, workers in industrial processing and food processing, farm workers, builders and painters and decorators, who may be exposed to detergents, harsh chemicals and irritants. For example, the formulation may be used as a skin barrier to latex allergens and may therefore find use in the treatment and/or prophylaxis of latex associated urticaria.

The formulation may be for use as a beneficial adjunct to hand hygiene practices / guidelines. Therefore the formulation may be used in conjunction with established hand washing materials.

The formulation may be for use as a skin barrier for humans or animals, including mammals such as domestic pets including dogs and cats, and farm animals including cows, pigs, sheep and horses. As used herein, the term "skin" refers to any external surface of a human or animal including the epidermis, mucous membranes, cornea, nails, teeth and hair.

The formulation may be administered under the supervision of a health care worker such as a doctor, or a veterinary surgeon.

The invention in a further aspect provides a barrier formulation of the invention for use in medicine, including veterinary medicine.

The formulation may be for use in the treatment and/or prophylaxis of skin conditions or infections. The skin conditions may be viral, bacterial, yeast or fungal. The skin conditions may include eczema, acne, psoriasis, dermatitis and athletes foot.

The formulation has been found to improve hand hygiene amongst health care workers, and therefore to act as an effective skin barrier. The formulation therefore may be for use in the treatment and/or prophylaxis of infection, and in particular in the treatment and/or prophylaxis of hospital acquired infections, such as Methicillin resistant *Staphylococcus aureus* (MRSA).

The formulation may also be used in the treatment and/or prophylaxis of the spread of infection by medical devices. The medical devices may include catheters used in medicine. In particular, the formulation may be used in conjunction with medical devices in the treatment and/or prophylaxis of renal disorders.

The formulation may also be used as a preparation to be applied to patients' skin before surgical operations, to treat and/or prevent infection before, during and after the surgical operations.

The formulation may also be used in the treatment of damaged skin. Therefore, the formulation may be used for wound healing such as for treating scrapes, grazes, cuts, scalds and bums formed in the skin. Without wishing to be bound by theory, it is believed that the formulation after application to the skin forms a barrier to the wound thereby promoting healing and preventing infection. The formulation may also be used in the treatment and/or prophylaxis of skin ulcers.

The formulation may be used in the treatment and/or prophylaxis of skin infections in animals. The formulation may be for use in the treatment and/or prophylaxis of mastitis infection of and/or the spread of mastitis infection between farm animals such as cattle. The formulation may be for use in the treatment and/or prophylaxis of teat sores in animals such as farm animals. The teat sores may be caused by Pseudocowpox or bovine herpes 2 viral infections.

The formulation may be for use to in the treatment and/or prophylaxis of mastitis, psoriasis, anthrax, ringworm and/or mud fever in animals. The formulation may be used to treat wounds, such as skin abrasions, scrapes, cuts, scalds and bums in the skin of animals.

The formulation may be administered under the supervision of a veterinary surgeon.

In a further aspect of the invention, there is provided the use of a barrier formulation of the invention in the manufacture of a medicament for use in the treatment and/or prophylaxis of the conditions mentioned herein.

The medicament may be for use in the treatment and/or prophylaxis of infections, such as skin infections and hospital acquired infections.

The medicament may be for use in the treatment and/or prophylaxis of skin infections in animals, such as mastitis infection and/or the spread of mastitis infection between farm animals, or teat sores in animals.

The silicone compound may be a silicone fluid. The silicone fluid may be dimethicone, a silicone emulsion, a dimethicone cross polymer, or a polydimethylsiloxane. The silicone fluid may be silicone fluid 200/100 CS. The formulation according to the invention may comprise from 0.1 to 10% by weight, from 0.5 to 5% by weight, or from 1% to 2% by weight of the silicone compound.

The formulation according to the invention may be in the form of a lotion in order to be easy to apply and to dispense, particularly in a hospital or veterinary environment. The viscosity of the formulation may be from 1 Pascal second (1000 cps) to 20 Pascal second (20000 cps). The viscosity of the formulation may be from 1 Pascal second (1000 cps) to 5 Pascal second (5000 cps).

The active ingredient may be included in the oil or water phase depending on in which phase it has greater solubility.

The active ingredient may be present in an amount from 1 to 5% by weight of the formulation, or from about 2% by weight.

The formulation according to the invention may optionally further comprise an emollient. The emollient may be selected from one of more of jojoba oil, avocado oil, sunflower oil, kukui nut oil sweet almond oil, coconut oil, apricot kernel oil, castor oil, emu oil, walnut oil, liquid paraffin and/or aloe vera. The formulation may further comprise a thickener. The thickener may be selected from one or more of cetyl palmitate or a carbomer or other gelling agent. The formulation may further comprise an excipient. The excipient may be selected from one or more of a glycol or alcohol, especially mono-propylene glycol. The formulation may further comprise a preservative. The preservative may be selected from one or more of a combination of esters of p-hydroxibenzoic acid (Nipastat (Trade Mark)), parabens, phenoxyethanol, a isothiazolinone, especially Phenonip (Trade Name), Nipasol (Trade Name), Nipagin (Trade Name), Euxyl K100 (Trade Name)). The formulation may further comprise a fragrance. The formulation may further comprise an emulsifier. The emulsifier may be selected from one or more stearate derivatives, for example glyceryl monostearate. The formulation may further comprise a neutralising agent. The neutralising agent may be selected from one or more of triethanolamine, sodium hydroxide or potassium hydroxide. The formulation may further comprise water.

In the method of the invention, the water phase may be added to the oil phase to obtain an oil-in-water emulsion, in which oil phase droplets are surrounded by water. It has been found that the formulation according to the invention has greater efficacy when it is in the form of an oil-in-water emulsion.

The addition of a neutralising agent to the mixture of oil phase and water phase is critical since the agent can only neutralise following the emulsification. This reaction not only adjusts the pH but also the viscosity of the finished product. It is particularly important when the formulation contains a carbomer as a thickener because the carbomer needs to be neutralised. A carbomer is a homopolymer of acrylic acid crosslinked with an allyl ether of pentaerythritol, and allyl ether of sucrose, or an allyl ether of propylene.

A suitable neutralising agent for use in the method of the invention is an alkaline agent. The neutralising agent may be triethanolamine, sodium hydroxide or potassium hydroxide.

The formulation may comprise the following ingredients in the ranges indicated:

| Ingredient | Range (wt%) |
|---|---|
| Castor oil | 0.5-3.0 |
| Stearic acid | 0.5-8.0 |
| Glycerol stearate | 0.1-3.0 |
| Cetyl palmitate | 0.1-2.0 |
| Silicone fluid | 0.1-10 |
| Nipastat | 0.1-0.5 |
| Jojoba oil | 0.1-0.5 |
| Liquid paraffin | 0.1-0.5 |
| Active ingredient | 0.5-10 |
| Water | 35-95 |
| Carbomer | 0.5-8.0 |
| Aloe vera | 0.1-2.0 |
| Monopropylene glycol | 2.0-15 |
| Triethanolamine | 0.1-2.0 |

The formulation may be prepared in the following steps:
1. Melting together the castor oil, stearic acid, glycerol stearate, cetyl palmitate, silicon fluid, jojoba oil, liquid paraffin and Nipastat.
2. Ensure the above a mixed together well and heat to between 70-80 °C.
3. In a separate vessel mix together the monopropylene glycol, triethanolamine, 50% of water and carbomer.
4. Heat to 65°C
5. Add the active component to (1) immediately before stage (6) and mix.
6. Add the oil phase from (1) to the water phase from (3) mixing well under high shear conditions
7. When fully mixed add aloe vera solution & stir
8. Add the remaining cold water and stir.
9. Leave overnight to stand and cool, then re-mix the total batch.

Preferred features of each aspect of the invention are as for each of the other aspects *mutatis mutandis.*

The invention will now be illustrated with reference to the following Examples which are not intended to limit the scope of protection obtained, and with reference to the accompanying figures, in which:
Figure 1 illustrates the mean CFU (colony forming units) from the hands of health care workers in the study and control arms discussed in Example 6;
Figure 2 illustrates the number of times hands were washed with non-medicated soap and water by health care workers in the control and study arms during the study period of Example 6; and
Figure 3 illustrates the total wearing time of gloves by health care workers in the control and study arms during the study period of Example 6.

### EXAMPLE 1

A barrier formulation having the ingredients listed in Table 1 was prepared as follows.

**TABLE 1**

| Materials | Amount/wt% | Phase |
|---|---|---|
| Castor oil | 2.00 | A |
| Stearic acid | 6.00 | |
| GMS SE | 2.00 | |
| Cetyl Palmitate | 1.00 | |
| Silicone fluid | 1.00 | |
| Nipastat | 0.20 | |
| Jojoba oil | 0.10 | |
| Liquid paraffin | 0.10 | |
| MPG | 10.00 | B |
| Triethanolamine | 1.55 | |
| Hot water @65°C | 39.65 | |
| Carbopol | 5.00 | |
| Triclosan | 0.50 | C |
| Aloe vera | 0.50 | D |
| Fragrance | 0.15 | |
| Cold water | 30.25 | E |

Wherein GMS SE is glyceryl stearate manufactured by Stepan Company, Nipastat is a preservative product containing methyl paraben, butyl paraben, ethyl paraben, propyl paraben and isobutylparaben manufactured by Clariant UK Ltd, MPG is the excipient monopropylene glycol. The silicone fluid is a dimethicone such as silicon fluid 200/100 CS.

The ingredients of phase A were melted together at temperature 60°C. The ingredients of phase B were mixed together using a Silverson High Shear Mixer. Phase C was added to phase A and stirred until dissolved. Phase B was then added to phase A. Phase D was then added and mixed using a Silverson High Shear Mixer. Phase E was also added while mixing.

### EXAMPLE 2

A barrier formulation having the ingredients listed in Table 2 was prepared as follows.

**TABLE 2**

| Materials | Amount/wt% | Phase |
|---|---|---|
| Castor oil | 2.0 | A |
| Stearic acid | 1.5 | |
| GMS SE | 0.5 | |
| Cetyl Palmitate | 0.5 | |
| Silicone fluid | 1.0 | |
| Nipastat | 0.2 | |
| Jojoba oil | 0.1 | |
| Liquid paraffin | 0.1 | |
| Triclosan | 1.0 | B |
| Hot water @ 60°C | 80.4 | C |
| Carbopol (5%) | 2.0 | |
| Aloe vera 10.1 | 0.5 | |
| MPG | 10.0 | |
| Triethanolamine | 0.2 | D |

The silicone fluid is a dimethicone, such as silicon fluid 200/100 CS.

The ingredients of phase A were melted together at temperature 70°C until the phase was clear. The ingredients of phase C were mixed together using a Silverson High Shear Mixer. Phase B was added to phase A and stirred until dissolved. Phase C was then added to phase A. Phase D was then added and mixed using a Silverson High Shear Mixer.

### EXAMPLE 3

A barrier formulation having the ingredients listed in Table 3 was prepared as follows.

**TABLE 3**

| Materials | Amount/wt% | Phase |
|---|---|---|
| Castor oil | 2.0 | A |
| Stearic acid | 1.5 | |
| GMS SE | 0.5 | |
| Cetyl Palmitate | 0.5 | |
| Silicone fluid | 1.0 | |
| Nipastat | 0.2 | |
| Jojoba oil | 0.1 | |
| Liquid paraffin | 0.1 | |
| Triclosan | 2.0 | B |
| MPG | 10.0 | C |
| Hot water @ 60°C | 79.4 | |
| Carbopol | 2.0 | |
| Aloe vera | 0.5 | |
| Triethanolamine | 0.2 | D |

The silicone fluid is a dimethicone, such as silicon fluid 200/100 CS.

The ingredients of phase A were melted together at temperature 65°C until clear. The ingredients of phase C were mixed together using a Silverson High Shear Mixer. Phase B was added to phase A and stirred until dissolved. Phase C was then added to phase A. Phase D was then added and mixed using a Silverson High Shear Mixer.

### EXAMPLE 4

The bactericidal efficacy of the formulation prepared in Example 2 was tested over a 4-hour period on the hands of volunteers. In addition, it was investigated whether repeated applications of soap and water, 4% (w/v) chlorhexidine gluconate (Hydrex) (Adams Healthcare, Leeds, UK) and 70% (vlv) isopropanol (Guest Medical, Kent, UK) to the hands, affected the efficacy of the cream. Five volunteers participated in the study, four of which applied the formulation prepared in Example 2 to their hands. A 2cm² area was designated on the left hand of each volunteer and 50 µl of *Staphylococcus epidermidis* NCTC 9865 at a concentration of approximately 1 x 10⁴ cfu/mL was applied and allowed to dry. *S. epidermidis* NCTC 9865 was selected as an indicator microorganism as it produces a red pigment when grown on nutrient agar.

At 1 hour, each 2cm² section was swabbed and cultured directly onto nutrient agar (Oxoid Ltd, Basingstoke, UK). Three volunteers then washed their hands thoroughly with either soap and water, 4% chlorhexidine gluconate or 70% isopropanol. All plates were incubated at 37°C in air for 48 hours. This process was repeated over a four-hour period at hourly intervals and on two consecutive days. The mean numbers of viable *S. epidermidis* NCTC 9865, recovered from the hands of the volunteers are shown in Table 4.

**TABLE 4**

| Colony forming units of *S. epidermidis* NCTC 9865 recovered from a 2cm² area of skin on the hands of volunteers. | | | | | | |
|---|---|---|---|---|---|---|
| Loading inoculum | Sampling time (hrs) | Control | No washing | Soap/water | 4% chlorhexidine gluconate | 70% isopropanol |
| 4.4x10² (0 hours) | 1 | 3.8x10² | 0 | 0 | 0 | 1 |
| 2.7x10² (1 hour) | 2 | 2x10² | 0 | 0 | 0 | 3 |
| 3.6x10² (2 hours) | 3 | 3.5x10² | 0 | 0 | 0 | 1 |
| 3.2x10² (3 hours) | 4 | 2.9x10² | 1 | 0 | 0 | 13 |

The results show that the formulation of Example 2 retained its antibacterial activity over a 4-hour period when challenged with between 3.2 x 10² and 4.4 x 10² cfu of *S. epidermidis* NCTC 9865. Three applications of soap and water, and 4% chlorhexidine gluconate to the hands during the study period did not affect the bactericidal activity of the formulation. Following a third application of 70% isopropanol, 13 cfu of *S. epidermidis* NCTC 9865 was recovered at 4 hours from one volunteer, however this still represented a 3.1 x 10² cfu reduction in viable microorganisms compared to the loading inoculum.

The results of this preliminary investigation demonstrate the usefulness of the formulation according to the invention as an effective anti-microbial barrier cream.

### EXAMPLE 5

A barrier formulation having the ingredients listed in Table 5 was prepared as follows.

**Table 5**

| Materials | Amount/wt% | Phase |
|---|---|---|
| Castor oil | 2.0 | A |
| Stearic acid | 1.5 | |
| GMS SE | 0.5 | |
| Cetyl Palmitate | 0.5 | |
| Silicone fluid 200/100 CS | 1.0 | |
| Nipastat | 0.2 | |
| Jojoba oil | 0.1 | |
| Liquid paraffin | 0.1 | |
| Triclosan | 2.0 | B |
| MPG | 10.0 | C |
| Water | 78.6 | |
| Carbopol 5% | 2.0 | |
| Aloe vera 10:1 | 0.5 | |
| Triethanolamine | 1.0 | D |

Silicon fluid 200/100 CS is a dimethicone.

The ingredients of phase A were melted together at temperature 65-80°C until clear. The ingredients of phase C were mixed together using a Silverson High Shear Mixer. Phase B was added to phase A and stirred until dissolved. Phase C was then added to phase A. Phase D was then added and mixed using a Silverson High Shear Mixer.

### EXAMPLE 6

The anti-microbial efficacy of the formulation of Example 5 was assessed in the clinical setting.

The acceptability of the formulation of Example 5 by health care workers (HCW) was investigated. In vitro and in vivo assessment of the formulation was undertaken in accordance with European standards currently used for assessing hygienic and surgical rubs and washes (prEN 12054 and prEN 12791).

### METHODS

### Health care worker recruitment

- HCW were recruited from two acute neurosurgical wards, a radiology department and two intensive care units within the University Hospital Birmingham NHS trust, UK.

### Study design

1) Ethical committee approval was obtained from South Birmingham Ethics Research Council prior to commencement of the study.
2) Computerised randomisation of HCW into study and control groups was performed.
3) All HCW washed their hands with non-medicated soap and water for 30 seconds and dried hands with sterile paper towels.
4) A 4cm² area of palm of the dominant hand of each HCW was sampled with a swab moistened with 0.9% (w/v) sterile saline for baseline microbial counts.
5) The study group then applied 0.5 ml of the formulation to their hands as recommended by manufacturer. The control group did not apply the formulation.
6) After 1 minute a 4cm² area from non-dominant hand was sampled as described previously.
7) HCW continued their work as normal. Other hand disinfectants, including alcohol hand rubs and chlorhexidine gluconate were not used during the study period. Any hand washes containing triclosan were removed from study areas one week prior to commencement of the study. However, during the study, HCW continued to wash their hands with non-medicated soap and water as per normal clinical practice.
8) After 1 hour and 3 hours further microbiological samples were taken from different areas of the palms of the dominant and non-dominant hand respectively as described previously.
9) Data on the number of times hands were washed with soap and the duration gloves were worn were collected with a standardised questionnaire.
10) HCW who had to use hand disinfection (alcohol/chlorhexidine) during the study due to clinical circumstance were not included in the evaluation
11) Acceptability of the formulation by HCW in the study group was investigated with a standardised questionnaire.

### Standardised questionnaires

HCW completed a standardised questionnaire at 1 and 3 hrs to indicate the following:
1. Number of times gloves were worn
2. Total time gloves were worn
3. Number of times hands were washed with non-medicated soap and water

In addition, HCW comprising the study group completed a further standardised questionnaire at 3 hrs which asked:
1. Did you experience any discomfort on your hands whilst wearing the formulation?
2. How did the formulation feel on your hands (good, acceptable, unacceptable)?
3. Did the presence of the formulation on your hands compromise manipulation technique?
4. Would you use the formulation as a part of your routine hand hygiene regime?
5. Overall comments?

### Quantitative microbial analysis

- Swabs taken at time 0,1 minute, 1 hour and 3 hours, were broken into 1 ml of neutralising Letheen broth (Merck, Germany) and vortexed for 30 seconds. Two x 0.5ml volumes of the sample were mixed thoroughly with 2 x15ml of cooled molten yeast extract agar (BioMerieux, UK) in a Petri dish. Culture plates were incubated at 37°C in air for 24 hours.
- Colony forming units (CFU) were determined and results expressed as mean CFU.
- The percentage reductions of mean CFU from the baseline bacterial counts were also determined.

### Statistical analysis

- Means were compared using the Mann-Whitney U test and p values of equal to, or less than 0.05 were considered significant.

### prEN 12054 and prEN 12791 (European standards for assessing Hygienic and Surgical hand rubs and washes)

- *In vitro* antibacterial efficacy of the formulation was assessed for anti-microbial activity against *Staphylococcus aureus* ATCC 6538, *Enterococcus hirae* ATCC 10541, *Pseudomonas aeruginosa* ATCC 15442 and *Escherichia coli* NCTC 10536 in accordance with prEN 12054. *In vivo* anti-microbial activity of the formulation was compared with 60% (v/v) n-propane (gold standard) in accordance with prEN 1279. Twenty laboratory volunteers participated in the assessment of the formulation in accordance to prEN 12791.

### Reassessment of antimicrobial activity of the formulation after 4 months

The formulation used for assessment of antimicrobial activity in accordance with prEN 12054 was allowed to stand at room temperature for 4-months. The antimicrobial efficacy of V was then reassessed in accordance with prEN 12054.

### RESULTS

### Health care worker recruitment

- One hundred and two HCW were recruited into the study. Fifty two HCW were randomised into the study arm, and 50 in the control arm.
- The recruited HCW were nurses (65), auxillaries (13), medical personnel (7), physio- or occupational therapists (9), radiographers (6) and porters (2).

### Quantitative and statistical analysis

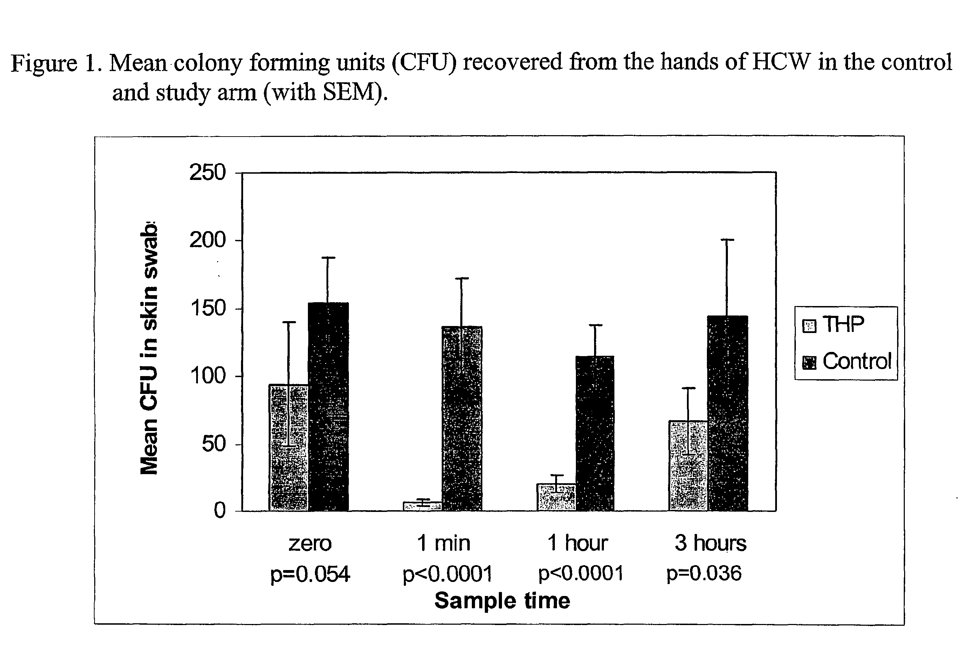

• The mean CFU obtained from the hands of HCW in the study and control arms are given in Figure 1. There was no significant difference in baseline mean CFU counts (p= 0.054). However significant reductions in mean CFU counts in the study arm after 1 minute (p<0.0001), 1 hour (p<0.0001) and 3 hours (p<0.036) were obtained (Table 6).

**Table 6. Percentage reduction in mean CFU on the hands of HCW in the control and study arms at 1 minute, 1 hour and 3 hours compared to baseline mean CFU counts.**

| Sample time | % mean reduction in CFU on the hands of HCW following application of the formulation | % mean reduction in CFU on the hands of HCW in the control arm |
|---|---|---|
| 1 minute | 93 | 11 |
| 1 hour | 78 | 26 |
| 3 hours | 29 | 6 |

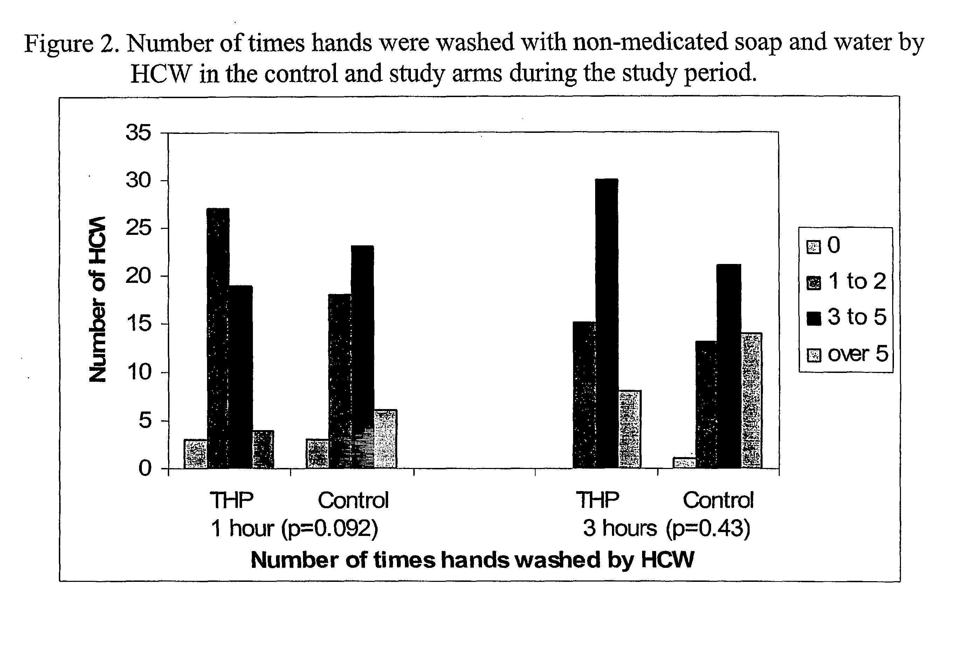

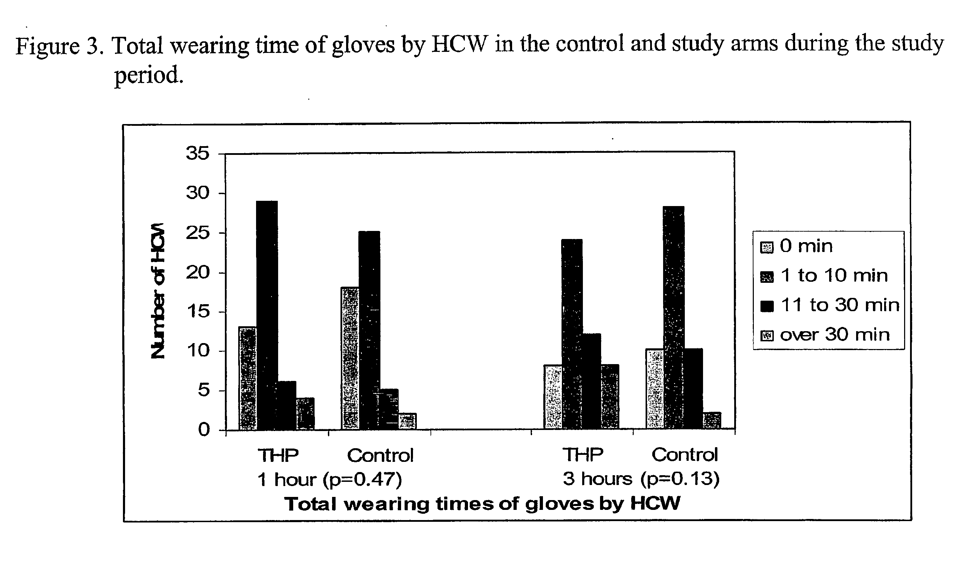

• The number of times hands were washed and duration gloves were worn did not differ significantly between the study group and controls (Figures 2 and 3). (hand washing at 1 hour p=0.092 and 3 hours p=0.43; Wearing of gloves after 1 hour p=0.47 and 3 hours p=0.13).

### Acceptability of the formulation by HCW

- None of the 52 HCW experienced any adverse reaction/skin irritation during the study period; 50 out of 52 (97%) reported that the formulation absorbed well into the hands and felt good/acceptable; all 52 HCW confirmed that the formulation did not compromise manipulation techniques; 49 out of 52 (94%) reported that they would use the formulation as a part of their daily hand hygiene regime. No adverse reactions to the formulation were reported during the study period.

### prEN 12054

- The formulation demonstrated rapid bactericidal activity against *Staphylococcus aureus* (ATCC 6538), *Enterococcus hirae* (ATCC 10541) and *Escherichia coli* (NCTC 10536) in accordance with prEN 12054. The formulation demonstrated no antimicrobial activity against *Pseudomonas aeruginosa* ATCC 15442 over the 10 min assessment period.

### prEN 12791

- There was a significantly higher mean reduction factor after 3 minutes (immediate) in the bacterial counts on the fingertips of 20 volunteers following application of 60% propanol compared with the formulation (p<0.0001). There was no significant difference in the mean reduction factors of bacterial counts following 3 hours (p=0.39).

### Reassessment of antimicrobial activity of the formulation after 4 months

- There was no significant difference in the antibacterial activity of the formulation in accordance with prEN 12054 following 4-months standing at room temperature (p= 0.155).

### CONCLUSIONS

- The mean bacterial count on the palms of the hands of HCW is reduced following application of the formulation.
- The bacterial counts on the palms of the hands of HCW remained significantly lower than those on the palms of HCW who did not apply the formulation for up to 3-hours in the routine clinical situation.
- There was no significant difference between the number of times hands were washed and duration gloves were worn between study and control groups. These results suggest that hand washing and wearing of gloves did not significantly account for differences observed in microbial loads on the hands of HCW who applied the formulation and those who did not. Furthermore, the results suggest that handwashing did not significantly reduce the activity of the formulation during the study period.
- the formulation was well accepted by HCW. the formulation absorbed well into the skin leaving it feeling well conditioned and did not compromise manipulation techniques in clinical practice. The majority of HCW reported that they would use the formulation as a part of their daily hand hygiene regime.
- In addition, several HCW who continued to use the formulation following the clinical evaluation reported improvements in the condition of their skin and a reduction in the presence of eczema and psoriasis (data not shown).
- the formulation demonstrated rapid bactericidal activity against *Staphylococcus aureus* (ATCC 6538), *Enterococcus hirae* (ATCC 10541) and *Escherichia coli* (NCTC 10536) in accordance with prEN 12054. The formulation did not demonstrate antibacterial activity against *Pseudomonas aeruginosa* ATCC 15442 and therefore did not conform to prEN 12054.
   Many strains of *Pseudomonas aeruginosa* are naturally resistant to many antimicrobials including triclosan and widespread, uncontrolled continued use of the formulation alone, may select for resistant strains in the clinical setting. Further *in vitro* testing of the formulation revealed that the preparation had rapid antimicrobial activity against microoraganisms not included in prEN 12054. Microorganisms included *Propionibacterium acnes* NCTC 797, Acinetobacter sp (wild strain) and Campylovbacter jejuni (wild strain).
- Assessment of the formulation in accordance with prEN 12791demonstrated that alcoholic hand disinfection results in a significantly higher immediate reduction in the bacterial flora on the fingertips of HCW with no significant difference in counts over time (3-hours). the formulation did not therefore conform to prEN12791.
- There was no significant difference in antimicrobial activity of the formulation following repeat testing after standing at room temperature for 4-months.
   These results suggest that the formulation retains antibacterial activity for at least 4-months at room temperature.
- the formulation may be a beneficial adjunct to current hand hygiene practices / guidelines and may facilitate in reducing cross-infection.
- Further in view of the formulation demonstrating rapid bactericidal activity against *Staphylococcus aureus,* the formulation finds use in the treatment and/or prevention and in particular the reduction of hospital acquired infections such as Methicillin resistant *Staphylococcus aureus* (MRSA).

### EXAMPLE 7

A 48 hour irritancy test of three barrier formulations detailed in Table 7 was performed on health care workers.

**Table 7**

| Formulation | Ingredients |
|---|---|
| 2% Triclosan | Castor oil |
| | Stearic acid |
| | GMS SE |
| | Cetyl palmitate |
| | Silicone fluid (dimethicone) |
| | Nipastat |
| | Jojoba oil |
| | Liquid paraffin |
| | MPG |
| | Triethanolamine |
| | Hot water |
| | Carbopol |
| | Triclosan |
| | Aloe vera |
| | Cold water |
| 1% Chlorhexidine | Castor oil Stearic acid |
| | GMS SE |
| | Cetyl palmitate |
| | Silicone fluid (dimethicone) |
| | Nipastat |
| | Jojoba oil |
| | Liquid paraffin |
| | MPG |
| | Triethanolamine |
| | Hot water |
| | Carbopol |
| | Chlorhexidine |
| | Aloe vera |
| | Cold water |
| 1% Tea Tree Oil | Castor oil Stearic acid |
| | GMS SE |
| | Cetyl palmitate |
| | Silicone fluid (dimethicone) |
| | Nipastat |
| | Jojoba oil |
| | Liquid paraffin |
| | MPG |
| | Triethanolamine |
| | Hot water |
| | Carbopol |
| | Tea Tree Oil |
| | Aloe vera |
| | Cold water |

26 male and female volunteers were recruited from a test panel. The mean age of the 26 subjects was 46 years, range 23-62 years. All subjects were healthy volunteers who had been given a medical examination before joining the trail. All volunteers were within the age range 18-65 years, and were healthy with no significant concurrent illnesses.

A number of exclusion criteria were used to select the volunteers for the study. These criteria are as follows:
◆ Pregnant or lactating females at the start of the study or females of reproductive age who did not agree to take contraceptive measures to avoid becoming pregnant during the study
◆ Subjects who had used any systemic or topical medication likely to interfere with the study
◆ Subjects who had used an experimental drug within 30 days or the study
◆ Subjects who had taken part in a study involving the test sites (skin of the lower back) within 8 weeks of the study
◆ Subjects with a history of, or evidence of alcohol or drug abuse.

Ethical approval for the study was obtained. All subjects gave their consent before starting the study.

0.1g of each of the formulations was applied to the lower part of the back between the lower coastal margin and the iliac crest, avoiding the area over the vertebral column using 12mm aluminium Finn chambers on Scanport® tape. The three test chambers were applied vertically in a single column of the chambers to one side of the back. Site number 1 was the upper test chamber and site number 3 was the lower test chamber.

The test materials were in continuous contact with the skin over a 48 hour period. After this period the test chambers were removed and the sites assessed using the following schedule:

| | |
|---|---|
| t = 0 hours | Apply material under occlusion |
| t = 24 hours | Remove, wait 10 minutes, assess sites, re-apply |
| t = 48 hours | Remove, assess sites |

The test sites were assessed visually for erythema at 24 and 48 hours, using a 0-6 ranking scale as follows:
Erythema
   0 = no reaction
   0.5 = slight, patchy erythema
   1= slight uniform erythema
   2 = moderate, uniform erythema
   3 = strong erythema
   4 = strong erythema, spreading outside patch
   5 = strong erythema, spreading outside patch with either swelling or vesiculation
   6 = severe reaction with erosion

If in addition to erythema other clinical signs of cutaneous irritation are observed the following letters were appended to the numerical score as follows:
OE - Oedema
V = Vesiculation
S = Scaling
C = Cracking or crazing
SC = Scabbing
P = Papules
SO = Reaction spreading outside area of application
G = Glazing
BS = Burning or Stinging reported by volunteer

The skin tolerance was determined by the number of grade 2 or greater skin reactions recorded. The following classification was used:

| Number of subjects with a grade 2 or stronger reaction | Skin tolerance 48 hour irritancy |
|---|---|
| None | Well tolerated |
| 1 | Tolerated well by most but not all subjects |
| 2 | Poorly tolerated |
| 3-4 | Very poorly tolerated |
| 5-9 | |
| More than 10 | |

The results of the study are provided below in Tables 8 to 10.

**Table 8**

| Assessment of Erythema - 24 hours | | | |
|---|---|---|---|
| Subject No. | Site 1 | Site 2 | Site 3 |
| 1 | 0 | 0 | 0 |
| 2 | 0 | 0 | 0 |
| 3 | 0 | 0 | 0 |
| 4 | 0 | 0 | 0 |
| 5 | 0 | 0 | 0 |
| 6 | 0 | 0 | 0 |
| 7 | 0 | 0 | 0 |
| 8 | 0 | 0 | 0 |
| 9 | 0 | 0 | 0 |
| 10 | 0 | 0 | 0 |
| 11 | 0 | 0 | 0 |
| 12 | 0 | 0 | 0 |
| 13 | 0 | 0 | 0 |
| 14 | 0 | 0 | 0 |
| 15 | 0 | 0 | 0 |
| 16 | 0 | 0 | 0 |
| 17 | 0 | 0 | 0 |
| 18 | 0 | 0 | 0 |
| 19 | 1 | 0 | 0 |
| 20 | 0.5 | 0 | 0 |
| 21 | 0 | 0 | 0 |
| 22 | 0 | 0 | 0 |
| 23 | 0 | 0 | 0 |
| 24 | 0.5 | 0 | 0 |
| 25 | 0 | 0 | 0 |
| 26 | 0 | 0 | 0 |

**Table 9**

| Assessment of Erythema - 48 hours | | | |
|---|---|---|---|
| Subject No. | Site 1 | Site 2 | Site 3 |
| 1 | 0 | 0 | 0 |
| 2 | 0 | 0 | 0 |
| 3 | 0 | 0 | 0 |
| 4 | 0 | 0.5 | 0 |
| 5 | 0 | 0 | 0 |
| 6 | 0 | 0 | 0 |
| 7 | 0.5 | 0 | 0.5 |
| 8 | 0 | 0 | 0 |
| 9 | 0.5 | 0 | 0 |
| 10 | 0 | 0 | 0 |
| 11 | 0 | 0 | 0 |
| 12 | 1 | 0 | 0 |
| 13 | 0 | 0 | 0 |
| 14 | 0 | 0 | 0 |
| 15 | 0 | 0 | 0 |
| 16 | 0.5 | 0 | 0 |
| 17 | 0 | 0 | 0 |
| 18 | 0.5 | 0 | 0.5 |
| 19 | 2 | 0 | 0 |
| 20 | 0.5 | 0 | 0 |
| 21 | 0 | 0 | 0 |
| 22 | 0 | 0 | 0 |
| 23 | 0.5 | 0 | 0 |
| 24 | 1 | 0 | 0.5 |
| 25 | 0 | 0 | 0 |
| 26 | 0 | 0 | 0 |

**Table 10**

| Summary of Results Results are expressed as the number of subjects reacting with a grade 1 or grade greater than 2 erythema | | |
|---|---|---|
| Formulation | Day 2 (24 hours) | Day 3 (48 hours) |
| 2% Triclosan | | |
| Score of 1 | 1 | 2 |
| Score of greater than 2 | 0 | 1 |

| 1% Chlorhexidine | | |
|---|---|---|
| Score of 1 | 0 | 0 |
| Score of greater than 2 | 0 | 0 |

| 1% Tea Tree Oil | | |
|---|---|---|
| Score of 1 | 0 | 0 |
| Score of greater than 2 | 0 | 0 |

The results therefore indicate that the formulation containing 2% Triclosan was tolerated well by most but not all subjects under the test conditions. The formulations containing 1% Chlorhexidine or 1% Tea Tree Oil were both well tolerated.

### EXAMPLE 8

A further study investigated the effects of a barrier formulation of the invention containing 0.5% Triclosan against bacteria generally found on farms, and the safety of the formulation in relation to milk quality. In particular the study was intended to determine whether when the formulation is applied to the hands of the milker and the teats of cows after milking, there is any effect on the incidence of mastitis in lactating dairy cows.

In the study the effect of the formulation on cow teat sores was also investigated. Teat sores can be caused by Pseudocowpox or Bovine Herpes 2 viral infections. Spread of infections can be a problem with regard to viral infections.

The formulation was applied to teat sores. The sores showed a more rapid recovery than previously expected. The study found that using the formulation on teats led to the teats becoming softer and more supple. In addition the spread of infection (both mastitis and teat lesions (warts and sores/ulcers)) between cows was reduced.

Contagious mastitis (*Staphlococcus aureus*) showed a marked downturn in the study. *Staphlococcus aureus* spreads via contact with milkers' hands and milking clusters. Cows identified as carriers were treated with antibiotics and had their teats wiped with the formulation pre and post milking. The study reported a reduction in overall somatic cell count (a rise in somatic cell count is often an indicator of sub-clinical infection with Staph aureus) and individual somatic cell count levels which may indicate that the formulation is helping to prevent the spread of *Staphlococcus aureus.*

In addition bacterial isolates from swabs of teats treated with the formulation, and milkers' hands, were found to be negative. This is therefore an indicator of the bactericidal activity of the formulation.

Further the use of the formulation on open wounds had remarkable results where conventional antibiotic preparations had failed.

Milkers who used the formulation on their hands regularly, for example before every milking - on hands and also on latex gloves, after morning/lunch breaks, and before any AI procedures, reported that the formulation reduced "pick up" of dirt and odour from the farm. In addition they reported that their hands were washed clean more easily than previously. Further the milkers indicated that their skin became more supple and softer to touch with a reduction in cracked skin and "ground in" dirt. Infections of cuts and scratches were also reported by milkers to be reduced.

## Claims

1. A barrier formulation which comprises an emulsion having at least an oil phase comprising a silicone compound and an aqueous phase, the viscosity of the formulation being 20 Pascal second (20000 cps) or less, one or more of an emollient, an excipient, a thickener, an emulsifier, a neutralising agent, a preservative, and water, and the formulation further consisting of triclosan as the only an active ingredient present in an amount from 0.5 to 10% by weight of the formulation.

2. A formulation as defined in claim 1 wherein the silicone compound is a silicone fluid which may be selected from dimethicone, a silicone emulsion, a dimethicone cross polymer, and a polydimethylsiloxane.

3. A formulation as defined in any one of the preceding claims, which comprises from 0.1 to 10% by weight, from 0.5 to 5% by weight, or from 1% to 2% by weight of the silicone compound.

4. A formulation as defined in any one of the preceding claims, wherein the active ingredient is present in an amount from 1 to 5% by weight, or about 2% by weight of the formulation.

5. A formulation as defined in any one of the preceding claims which further comprises a fragrance.

6. A formulation as defined in any one of the preceding claims, wherein the formulation comprises the following ingredients in the ranges indicated:
| Ingredient | Range (wt%) |
|---|---|
| Castor oil | 0.5-3.0 |
| Stearic acid | 0.5-8.0 |
| Glycerol stearate | 0.1-3.0 |
| Cetyl palmitate | 0.1-2.0 |
| Silicone fluid | 0.1-10 |
| Nipastat | 0.1-0.5 |
| Jojoba oil | 0.1-0.5 |
| Liquid paraffin | 0.1-0.5 |
| Active ingredient | 0.5-10 |
| Water | 35-95 |
| Carbomer | 0.5-8.0 |
| Aloe vera | 0.1-2.0 |
| Monopropylene glycol | 2.0-15 |
| Triethanolamine | 0.1-2.0 |

7. A barrier formulation as defined in any one of the preceding claims, for use as a skin barrier for humans or animals.

8. A barrier formulation as defined in any one of claims 1 to 6, for use in medicine, including veterinary medicine.

9. Use of a formulation as defined in any one of claims 1 to 6, in the manufacture of a medicament for use in the treatment and/or prophylaxis of infections.

10. Use of a formulation as defined in claim 9, wherein the medicament is for use in the treatment and/or prophylaxis of skin infections.

11. Use of a formulation as defined in claim 9, wherein the medicament is for use in the treatment and/or prophylaxis of hospital acquired infections.

12. Use of a formulation as defined in claims 9 or 10, wherein the medicament is for use in the treatment and/or prophylaxis of skin infections in animals.

13. Use of a formulation as defined in claim 12, wherein the medicament is for use in the treatment and/or prophylaxis of mastitis infection and/or the spread of mastitis infection between farm animals.

14. Use of a formulation as defined in claim 12, wherein the medicament is for use in the treatment and/or prophylaxis of teat sores in animals

15. A method of manufacturing a formulation claimed in any one of claims 1 to 8 comprising an emulsion having at least an oil phase comprising a silicone compound and an aqueous phase, the viscosity of the formulation being 20 pascal second (20,000 cps) or less, and the formulation further consisting of triclosan as the only active ingredient present in an amount from 0.5 to 10% by weight of the formulation, which method comprises the steps of:
(a) preparing an oil phase containing a silicone compound;
(b) preparing an aqueous phase;
(c) mixing the oil phase and the aqueous phase together;
(d) neutralising the mixture with a neutralising agent.

16. A method as defined in claim 15 wherein the water phase is added to the oil phase to obtain an oil-in-water emulsion.

## Patentansprüche

1. Barriereformulierung, umfassend eine Emulsion mit mindestens einer Ölphase, umfassend eine Silikonverbindung und eine wässerige Phase, wobei die Viskosität der Formulierung 20 Pascalsekunden (20 000 cps) oder weniger beträgt, einen oder mehrere Weichmacher, einen Arzneimittelträger, ein Verdickungsmittel, einen Emulgator, ein Neutralisierungsmittel, ein Konservierungsmittel und Wasser, wobei die Formulierung des Weiteren aus Triclosan als einzig aktivem Inhaltsstoff besteht, der in einer Menge von 0,5 bis 10 %-Masse der Formulierung vorhanden ist.

2. Formulierung gemäß Anspruch 1, wobei die Silikonverbindung eine Silikonflüssigkeit ist, die aus Dimethicon, einer Silikonemulsion, einem Dimethicon-Kreuzpolymer und einem Polydimethylsiloxan ausgewählt werden kann.

3. Formulierung gemäß einem der vorstehenden Ansprüche, die 0,1 bis 10 %-Masse, 0,5 bis 5 %-Masse oder 1% bis 2 %-Masse der Silikonverbindung umfasst.

4. Formulierung gemäß einem der vorstehenden Ansprüche, wobei der aktive Inhaltsstoff in einer Menge von 1 bis 5 %-Masse oder ca. 2 %-Masse der Formulierung vorhanden ist.

5. Formulierung gemäß einem der vorstehenden Ansprüche, die des Weiteren einen Duftstoff umfasst.

6. Formulierung gemäß einem der vorstehenden Ansprüche, wobei die Formulierung die folgenden Inhaltsstoffe in den angegebenen Bereichen umfasst:
| Inhaltsstoff | Bereich (%-Masse) |
|---|---|
| Rizinusöl | 0,5 - 3,0 |
| Stearinsäure | 0,5 - 8,0 |
| Glycerinstearat | 0,1 - 3,0 |
| Cetylpalmitat | 0,1 - 2,0 |
| Silikonflüssigkeit | 0,1 - 10 |
| Nipastat | 0,1 - 0,5 |
| Jojobaöl | 0,1 - 0,5 |
| Flüssiges Paraffin | 0,1 - 0,5 |
| Aktiver Inhaltsstoff | 0,5 - 10 |
| Wasser | 35 - 95 |
| Carbomer | 0,5 - 8,0 |
| Aloe vera | 0,1 - 2,0 |
| Monopropylenglykol | 2,0 - 15 |
| Triethanolamin | 0,1 - 2,0 |

7. Barriereformulierung gemäß einem der vorstehenden Ansprüche zur Verwendung als Hautbarriere für Menschen und Tiere.

8. Barriereformulierung gemäß einem der Ansprüche 1 bis 6 zur Verwendung in der Medizin einschließlich Veterinärmedizin.

9. Verwendung einer Formulierung gemäß einem der Ansprüche 1 bis 6 zur Herstellung eines Medikaments zur Verwendung in der Behandlung und/oder Prophylaxe von Infektionen.

10. Verwendung einer Formulierung gemäß Anspruch 9, wobei das Medikament zur Verwendung in der Behandlung und/oder Prophylaxe von Hautinfektionen bestimmt ist.

11. Verwendung einer Formulierung gemäß Anspruch 9, wobei das Medikament zur Verwendung in der Behandlung und/oder Prophylaxe von Krankenhausinfektionen bestimmt ist.

12. Verwendung einer Formulierung gemäß Anspruch 9 oder 10, wobei das Medikament zur Verwendung in der Behandlung und/oder Prophylaxe von Hautinfektionen bei Tieren bestimmt ist.

13. Verwendung einer Formulierung gemäß Anspruch 12, wobei das Medikament zur Verwendung in der Behandlung und/oder Prophylaxe von Mastitis-Infektionen und/oder der Übertragung einer Mastitis-Infektion zwischen Nutztieren bestimmt ist.

14. Verwendung einer Formulierung gemäß Anspruch 12, wobei das Medikament zur Verwendung in der Behandlung und/oder Prophylaxe von wunden Zitzen bei Tieren bestimmt ist.

15. Verfahren zur Herstellung einer Formulierung gemäß einem der Ansprüche 1 bis 8, umfassend eine Emulsion mit mindestens einer Ölphase, umfassend eine Silikonverbindung, und einer wässerigen Phase, wobei die Viskosität der Formulierung 20 Pascalsekunden (20 000 cps) oder weniger beträgt, wobei die Formulierung des Weiteren aus Triclosan als einzigem aktivem Inhaltsstoff besteht, der in einer Menge von 0,5 bis 10 %-Masse der Formulierung vorhanden ist, wobei das Verfahren die folgenden Schritte umfasst:
(a) Herstellung einer Ölphase, die eine Silikonverbindung enthält;
(b) Herstellung einer wässerigen Phase;
(c) Mischen der Ölphase und der wässerigen Phase;
(d) Neutralisierung der Mischung mit einem Neutralisierungsmittel.

16. Verfahren gemäß Anspruch 15, wobei die wässerige Phase zur Ölphase zugesetzt wird, um eine Öl-in-Wasser-Emulsion zu erhalten.

## Revendications

1. Formulation barrière qui comprend une émulsion ayant au moins une phase huileuse comprenant un composé de silicone et une phase aqueuse, la viscosité de la formulation étant de 20 Pascal-secondes (20 000 cps) ou moins, un ou plusieurs éléments parmi un émollient, un excipient, un épaississant, un émulsionnant, un agent neutralisant, un conservateur, et de l'eau, et la formulation étant en outre constituée de triclosan comme seul ingrédient actif présent dans une quantité de 0,5 à 10 % en poids de la formulation.

2. Formulation selon la revendication 1, dans laquelle le composé de silicone est un fluide de silicone qui peut être choisi parmi la diméthicone, une émulsion de silicone, un polymère croisé de diméthicone, et un polydiméthylsiloxane.

3. Formulation selon l'une quelconque des revendications précédentes, qui comprend de 0,1 à 10 % en poids, de 0,5 à 5 % en poids, ou de 1 % à 2 % en poids du composé de silicone.

4. Formulation selon l'une quelconque des revendications précédentes, dans laquelle l'ingrédient actif est présent dans une quantité comprise entre 1 et 5 % en poids, ou environ 2 % en poids de la formulation.

5. Formulation selon l'une quelconque des revendications précédentes, qui comprend en outre une fragrance.

6. Formulation selon l'une quelconque des revendications précédentes, dans laquelle la formulation comprend les ingrédients suivants, dans les gammes indiquées :
| Ingrédient | Gamme (% en poids) |
|---|---|
| Huile de ricin | 0,5-3,0 |
| Acide stéarique | 0,5-8,0 |
| Stéarate de glycérol | 0,1-3,0 |
| Palmitate de cétyle | 0,1-2,0 |
| Fluide de silicone | 0,1-10 |
| Nipastat | 0,1-0,5 |
| Huile de jojoba | 0,1-0,5 |
| Paraffine liquide | 0,1-0,5 |
| Ingrédient actif | 0,5-10 |
| Eau | 35-95 |
| Carbomère | 0,5-8,0 |
| Aloé vera | 0,1-2,0 |
| Monopropylène glycol | 2,0-15 |
| Triéthanolamine | 0,1-2,0 |

7. Formulation barrière selon l'une quelconque des revendications précédentes, à utiliser comme barrière dermique pour les humains ou animaux.

8. Formulation barrière selon l'une quelconque des revendications 1 à 6, à utiliser dans la médecine, y compris la médecine vétérinaire.

9. Utilisation d'une formulation selon l'une quelconque des revendications 1 à 6, dans la fabrication d'un médicament à utiliser dans le traitement et/ou la prophylaxie d'infections.

10. Utilisation d'une formulation selon la revendication 9, dans laquelle le médicament est destiné à être utilisé dans le traitement et/ou la prophylaxie d'infections de la peau.

11. Utilisation d'une formulation selon la revendication 9, dans laquelle le médicament est destiné à être utilisé dans le traitement et/ou la prophylaxie d'infections nosocomiales.

12. Utilisation d'une formulation selon les revendications 9 ou 10, dans laquelle le médicament est destiné à être utilisé dans le traitement et/ou la prophylaxie des infections de la peau chez les animaux.

13. Utilisation d'une formulation selon la revendication 12, dans laquelle le médicament est destiné à l'utilisation dans le traitement et/ou la prophylaxie d'une infection par mastite et/ou le développement de l'infection par mastite entre des animaux de la ferme.

14. Utilisation d'une formulation selon la revendication 12, dans laquelle le médicament est destiné à être utilisé dans le traitement et/ou la prophylaxie des ulcères des seins chez les animaux.

15. Procédé de fabrication d'une formulation selon l'une quelconque des revendications 1 à 8, comprenant une émulsion ayant au moins une phase huileuse comprenant un composé de silicone et une phase aqueuse, la viscosité de la formulation étant de 20 pascal-secondes (20 000 cps) ou moins, et la formulation étant en outre constituée de triclosan comme seul ingrédient actif présent, dans une quantité de 0,5 à 10 % en poids de la formulation, ledit procédé comprenant les étapes consistant à :
(a) préparer une phase huileuse contenant un composé de silicone ;
(b) préparer une phase aqueuse ;
(c) mélanger la phase huileuse et la phase aqueuse ensemble ;
(d) neutraliser le mélange avec un agent neutralisant.

16. Procédé selon la revendication 15, dans lequel la phase aqueuse est ajoutée à la phase huileuse pour obtenir une émulsion huile-en-eau.
